# FASCICULE DE BREVET EUROPEEN

(11) **EP 1 262 202 B1**
(45) Date de publication et mention de la délivrance du brevet: **09.04.2014**
(21) Numéro de dépôt: 02291279.4
(22) Date de dépôt: 24.05.2002
(51) Int. Cl.: A61M 1/02

(54) **Ligne de prélèvement du sang placentaire comprenant une poche de rinçage**
Set mit Spülflüssigkeitsbeutel zur Gewinnung von Plazentablut
Collection set for placental blood with rinsing fluid bag

(30) Priorité: 01.06.2001 FR 0107262
(43) Date de publication de la demande: 04.12.2002
(73) Titulaire: MACO PHARMA, 59420 Mouvaux (FR)
(72) Inventeur: Deverre, Frédéric, 59000 Lille (FR)
(74) Mandataire: Herrou, Nathalie

(56) Documents cités:
- EP-A- 0 987 034
- US-A- 4 850 995
- US-A- 5 879 318
- US-B1- 6 207 063

## Description

L'invention concerne une ligne de prélèvement du sang placentaire ainsi qu'un procédé de prélèvement à l'aide de cette ligne.

Elle s'applique typiquement au recueil des cellules souches hématopoïétiques (CSH) présentes dans le sang placentaire. Les CSH sont au coeur des nouvelles thérapies cellulaires, notamment sous forme de greffe à des patients atteints de maladies hématologiques congénitales ou acquises comme le cancer ou les leucémies. En effet, de récentes études scientifiques ont montré que la greffe de CSH issues du sang placentaire est un traitement thérapeutique de grande qualité du fait notamment d'une grande prolifération des cellules injectées et du nombre de rejets bien inférieur aux autres sources de cellules.

Pour prélever le sang placentaire, on connaît, notamment du document US-5 879 318, des systèmes comprenant une poche de recueil associée à une ou deux aiguilles de prélèvement.

Ce type de systèmes présente notamment l'inconvénient de ne permettre le prélèvement que d'une quantité réduite de sang placentaire (environ 100 ml) et donc de CSH, ce qui ne permet pas le greffage des adultes.

En outre, lorsque le prélèvement n'est pas réalisé de façon optimale, le volume prélevé devient insuffisant pour son utilisation dans le cadre d'une thérapie cellulaire.

Les documents WO-83/01573 et US-4 407 660 décrivent des systèmes à poches pour plasmaphérèse dans lesquels un kit de transfusion comprenant une poche contenant une solution saline, une chambre compte-goutte et un filtre est ou peut être connecté à la tubulure associée à l'aiguille de prélèvement. La solution saline est injectée au donneur pour rincer les tubulures et conserver la perméabilité de l'aiguille en attendant la réinjection des globules rouges.

L'invention vise donc à remédier à ces inconvénients en proposant notamment une ligne de prélèvement de sang placentaire qui permet, en circuit clos et de façon stérile, de prélever simplement une quantité de sang plus importante afin d'offrir aux cliniciens des doses thérapeutiques de CSH supérieures et ouvrir ainsi aux CSH issues de sang de cordon la voie pour les greffes d'adultes.

A cet effet, et selon un premier aspect, l'invention propose une ligne de prélèvement du sang placentaire telle que revendiquée, comprenant une poche de recueil en communication fluidique, par l'intermédiaire d'une première tubulure associée à un orifice d'entrée de la poche, avec au moins une aiguille de prélèvement, ladite ligne comprenant en outre une poche contenant une solution de rinçage qui est connectée ou qui est destinée à être connectée par l'intermédiaire d'une deuxième tubulure à ladite première tubulure.

Selon un deuxième aspect, l'invention propose un procédé de prélèvement de sang placentaire tel que revendiqué à l'aide d'une telle ligne, comprenant les étapes prévoyant de :
- éventuellement connecter la poche contenant la solution de rinçage ;
- réaliser une première ponction d'une veine du cordon ombilical ou du placenta avec une aiguille ;
- recueillir du sang placentaire dans la poche de recueil ;
- vider, par l'intermédiaire de l'aiguille piquée, la poche contenant la solution de rinçage à l'intérieur du cordon et du placenta ;
- réaliser une deuxième ponction de la veine du cordon ou du placenta avec la même ou avec une autre aiguille ;
- recueillir, dans la poche de recueil, du sang placentaire éventuellement additionné de solution de rinçage ;
- éventuellement introduire la solution anticoagulante et/ou de conservation dans la poche de recueil.

D'autres objets et avantages de l'invention apparaîtront au cours de la description qui suit en référence aux dessins annexés.
La figure 1 représente, en vue schématique de face, un premier mode de réalisation d'une ligne de prélèvement suivant l'invention.
La figure 2 représente, en vue schématique de face, un deuxième mode de réalisation d'une ligne de prélèvement suivant l'invention.
La figure 3 représente, en vue schématique de face, un troisième mode de réalisation d'une ligne de prélèvement suivant l'invention.
La figure 4 représente, en vue schématique de face, un quatrième mode de réalisation d'une ligne de prélèvement suivant l'invention.

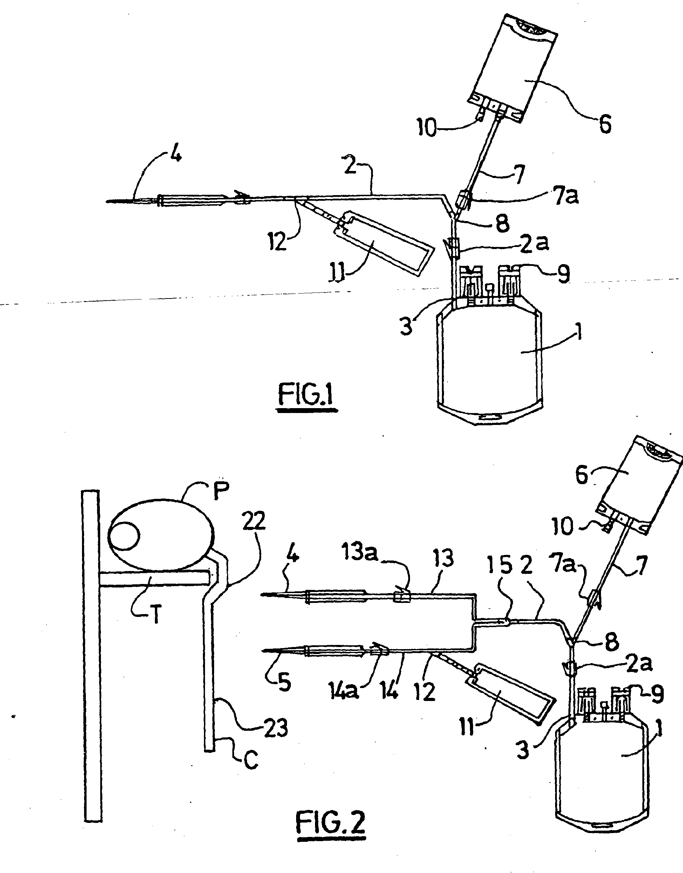

Sur les figures 1 à 4, est représentée une ligne de prélèvement comprenant une poche de recueil 1 en communication fluidique, par l'intermédiaire d'une première tubulure 2 associée à un orifice d'entrée 3 de la poche 1, avec au moins une aiguille de prélèvement 4, 5.

Cette ligne est typiquement destinée à permettre le prélèvement du sang placentaire. A cet effet, l'aiguille 4, 5 est piquée dans une veine du cordon ombilical C afin de provoquer l'écoulement par gravité du sang placentaire dans la poche de recueil 1.

Selon une pratique, on attend l'expulsion du placenta P et, après séparation du cordon C de l'enfant, on place le placenta P sur une tablette de travail T de laquelle le cordon C est laissé pendant afin de permettre le recueil du sang par gravité.

La ligne comprend en outre une poche 6 dite de rinçage contenant une solution de rinçage, par exemple formée d'une solution aqueuse de chlorure de sodium ou de toute autre solution inerte pour le sang. Comme expliqué dans la suite de la description, cette poche 6 permet de prélever une quantité plus importante de sang placentaire qu'avec les lignes de prélèvement de l'art antérieur.

Typiquement, la poche de rinçage 6 contient une quantité de solution de rinçage comprise entre 30 ml et 2 I de sorte à permettre le rinçage du cordon C et du placenta P.

Dans une première variante (représentée sur les figures), la poche de rinçage 6 est pourvue d'une deuxième tubulure 7 qui est connectée, par l'intermédiaire d'une première jonction 8, par exemple en T ou en Y, à la première tubulure 2.

Dans une deuxième variante non représentée, la poche de rinçage 6 est destinée, par exemple juste avant le prélèvement, à être associée à la ligne. A cet effet, la deuxième tubulure 7 est pourvue de moyens de connexion, par exemple stérile ou sous la forme d'un luer ou d'un perforateur, avec la première tubulure 2.

Dans ces deux variantes, le prélèvement est assuré en circuit clos et de façon stérile afin d'éviter toute contamination bactérienne du sang recueilli.

Les poches sont typiquement formées de deux feuilles de matière plastique assemblées, par exemple par soudure, sur leur périphérie extérieure de sorte à définir un volume intérieur et des orifices d'entrée et/ou de sortie.

Les feuilles sont formées en matériau plastique souple, biocompatible, soudable et stérilisable, par exemple en polychlorure de vinyle.

Les orifices d'entrée et/ou de sortie sont agencés pour recevoir de façon étanche des tubulures ou sont obturés de façon étanche et stérile par un dispositif 9 qui peut être déchiré dans le cas où l'opérateur souhaiterait les utiliser.

La poche de rinçage 6 peut également comprendre un port d'injection 10 afin de permettre l'injection éventuelle d'une substance dans la solution de rinçage.

Dans les modes de réalisation représentés, la ligne comprend en outre une poche 11 contenant une solution anticoagulante et/ou de conservation pour le sang prélevé, par exemple de type CPD.

Dans le premier mode de réalisation représenté sur la figure 1, la poche 11 est connectée, par l'intermédiaire d'une troisième jonction 12 prévue à proximité de l'aiguille 4, sur la première tubulure 2.

Bien que cette réalisation permette, en fin de prélèvement, un rinçage de la première tubulure 2 avec la solution anticoagulante et/ou de conservation et donc une augmentation de la quantité de sang prélevé, on peut prévoir que la poche de recueil 1 contienne de fabrication la solution anticoagulante et/ou de conservation.

On décrit ci-dessous les trois modes de réalisation représentés respectivement sur les figures 2 à 4, dans lesquels la ligne comprend une première 4 et une deuxième 5 aiguilles de prélèvement.

Comme on le verra dans la suite de la description, cette réalisation permet une manipulation plus aisée de la ligne lors du prélèvement.

Les deux aiguilles 4, 5 sont en communication fluidique avec respectivement une troisième 13 et une quatrième 14 tubulures, lesdites troisième 13 et quatrième 14 tubulures étant associées à la première tubulure 2 par l'intermédiaire d'une deuxième jonction 15.

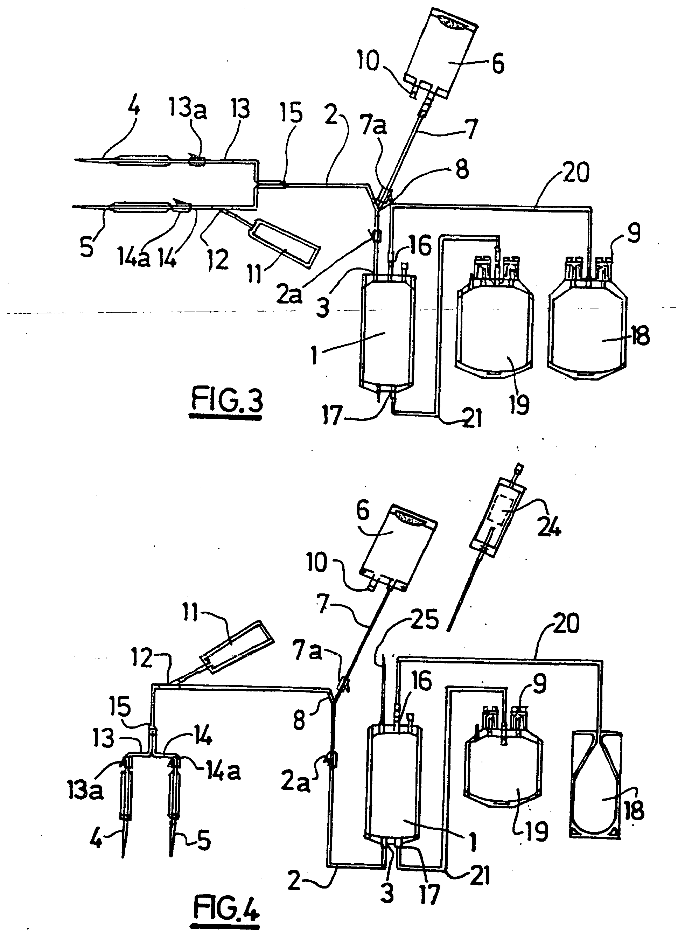

Dans les deuxième et troisième modes de réalisation (figure 2 et 3), la poche 11 contenant la solution anticoagulante et/ou de conservation est associée à la quatrième tubulure 14 alors que dans le quatrième mode de réalisation (figure 4) elle est associée à la première tubulure 2 à proximité de la deuxième jonction 15. Ces deux réalisations permettent de laver, avec la solution anticoagulante et/ou de conservation, les tubulures ayant servi à recueillir du sang placentaire.

Dans les troisième et quatrième modes de réalisation, la poche de recueil 1 comprend deux orifices de sortie 16, 17 prévus respectivement sur la partie supérieure et sur la partie inférieure de la poche 1, une première 18 et une deuxième poches 19 étant prévues pour être en communication fluidique par l'intermédiaire d'une tubulure 20, 21 avec respectivement l'un de ces orifices 16, 17.

Les tubulures sont souples, sécables et soudables et comprennent chacune des moyens sélectifs d'ouverture/fermeture de la communication fluidique à travers la tubulure, par exemple formés de clamp 2a, 7a, 13a et 14a.

On décrit ci-dessous un procédé de prélèvement de sang placentaire à l'aide d'une ligne suivant l'invention tel que revendiqué.

Dans une première étape, et dans le cas où elle n'est pas connectée de fabrication à la ligne, on connecte de façon stérile la poche contenant la solution de rinçage 6.

Ensuite, après par exemple disposition du placenta P sur une tablette de travail (voir figure 2) et ligature de l'extrémité libre du cordon C, on réalise une première ponction d'une veine ombilicale ou du placenta P. Par exemple, on pique une partie supérieure 22 d'une veine du cordon ombilical avec une aiguille 4. Par le terme supérieur, on entend à proximité du placenta P.

Dans les modes de réalisation où deux aiguilles 4, 5 sont prévues, c'est la première aiguille supérieure 4 qui est piquée en premier (voir figure 2).

Lors de l'ouverture des clamps 13a et 2a, du sang placentaire s'écoule alors par gravité dans la poche de recueil 1.

Une fois que cet écoulement est terminé le clamp 2a est fermé et le clamp 7a est ouvert afin de vider, par l'intermédiaire de l'aiguille 4 piquée, la poche 6 contenant la solution de rinçage à l'intérieur du cordon C et du placenta P.

Cette injection de la solution de rinçage permet de rincer la veine ombilicale et donc de récupérer une quantité plus importante de sang placentaire. En outre, elle permet d'augmenter la pression à l'intérieur du placenta P ce qui augmente d'autant l'expulsion du sang qu'il contient.

Dans une étape ultérieure, on réalise une deuxième ponction de la veine ombilicale ou du placenta P. Par exemple, on pique une partie inférieure 23 de la veine du cordon C, c'est à dire une partie éloignée du placenta P.

Dans le premier mode de réalisation, l'aiguille 4 est d'abord retirée de la partie supérieure 22 pour être piquée dans la partie inférieure 23, alors que, dans les trois autres modes de réalisation, c'est la deuxième aiguille 5 qui est piquée dans la partie inférieure 23.

L'utilisation de deux aiguilles 4, 5 permet une manipulation plus aisée de la ligne de transfusion en agençant la longueur des troisième 13 et quatrième 14 tubulures de sorte à permettre la piqûre des première 4 et deuxième 5 aiguilles respectivement dans la partie supérieure 22 et inférieure 23 de la veine ombilicale.

Dans d'autres modes de réalisations, la première et la deuxième ponctions peuvent être réalisées sensiblement au même endroit ou à des positions relatives différentes. En particulier, et dans le cas du premier mode de réalisation, l'aiguille 4 peut être laissée piquée au même endroit pour la première et la deuxième ponctions.

En ouvrant les clamps 14a, 2a et en fermant le clamp 7a, on peut alors recueillir, dans la poche de recueil 1, du sang placentaire éventuellement additionné de solution de rinçage.

Le fait de prévoir de piquer une partie supérieure 22 de la veine pour injecter la solution de rinçage permet notamment d'améliorer la récupération du sang contenu dans le placenta P, alors que le recueil du sang additionné de la solution de rinçage est prévue dans une partie inférieure 23 afin d'augmenter la quantité de sang prélevé en augmentant notamment la pression d'écoulement.

Des essais ont montré qu'il était possible de récupérer jusqu'à deux fois plus de sang ombilical que dans les procédés n'utilisant pas de solution de rinçage.

Dans le cas où elle n'est pas présente de fabrication dans la poche de recueil 1, la solution anticoagulante et/ou de conservation est ensuite introduite dans la poche de recueil, en fermant le clamp 14a, afin de permettre le lavage de la tubulure 2, 14 ayant servi à recueillir du sang placentaire.

Dans une étape ultérieure la poche de recueil 1 peut être désolidarisée de la ligne de prélèvement, par exemple par coupure et soudure de la première tubulure 2 à proximité de l'orifice d'entrée 3.

La poche 1 contenant le sang placentaire peut alors être traitée, par exemple par centrifugation, pour permettre de réduire le volume contenant les CSH.

En effet, sous l'effet d'une centrifugation adaptée, le sang placentaire se sépare en trois couches comprenant respectivement le plasma et éventuellement la solution de rinçage, les leucocytes et les CSH qui ont sensiblement la même densité, et les globules rouges.

A cet effet, dans les troisième et quatrième modes de réalisation, une première et une deuxième poches 18, 19 sont associées respectivement à un orifice de sortie supérieur 16 et inférieur 17 de la poche de recueil 1 pour permettre, par pression de la poche de recueil 1, la récupération de respectivement la couche de plasma et le concentré de globules rouges à l'intérieur de celles-ci.

Ainsi, après cette étape et éventuellement désolidarisation des première et deuxième poches 18, 19, par exemple par coupure et soudure des tubulures 20, 21, la poche de recueil 1 ne contient plus que la couche intermédiaire comprenant les leucocytes et les CSH.

Dans le quatrième mode de réalisation (figure 4), une troisième poche 24 est prévu pour être connectée de façon stérile à un orifice de sortie 25 de la poche de recueil 1 afin de récupérer son contenu.

Dans tous les modes de réalisation, le sang prélevé ou la couche intermédiaire peut, après ajout d'une solution de cryopréservation tel que DMSO et solution protéique (albumine, HEA), être conservée par congélation ou être traitée de façon conventionnelle afin de récupérer les CSH.

## Revendications

1. Ligne de prélèvement du sang placentaire, comprenant une poche de recueil (1) en communication fluidique, par l'intermédiaire d'une première tubulure (2) associée à un orifice d'entrée (3) de la poche (1), avec une première (4) et une deuxième (5) aiguilles de prélèvement, lesdites aiguilles de prélèvement étant en communication fluidique avec respectivement une troisième (13) et une quatrième (14) tubulures, lesdites troisième (13) et quatrième (14) tubulures étant associées à la première tubulure (2) par l'intermédiaire d'une jonction (15),
ladite ligne étant **caractérisée en ce qu'**elle comprend en outre une poche (6) contenant une solution de rinçage qui est connectée ou qui est destinée à être
connectée par l'intermédiaire d'une deuxième tubulure (7) à ladite première tubulure (2).

2. Ligne de prélèvement selon la revendication 1, **caractérisée en ce que** les première (2) et deuxième (7) tubulures sont connectées par l'intermédiaire d'une première jonction (8).

3. Ligne de prélèvement selon l'une quelconque des revendications 1 à 2, **caractérisée en ce que** les tubulures comprennent chacune des moyens sélectifs d'ouverture/fermeture de la communication fluidique à travers la tubulure.

4. Ligne de prélèvement selon l'une quelconque des revendications 1 à 3, **caractérisée en ce que** la poche de recueil (1) comprend une solution anticoagulante et/ou de conservation.

5. Ligne de prélèvement selon l'une quelconque des revendications 1 à 3, **caractérisée en ce qu'**elle comprend en outre une poche (11) contenant une solution anticoagulante et/ou de conservation, ladite poche (11) étant connectée, par l'intermédiaire d'une troisième jonction (12) prévue à proximité d'une aiguille de prélèvement (4, 5), sur la première (2) ou sur la quatrième (14) tubulure.

6. Ligne de prélèvement selon l'une quelconque des revendications 1 à 5, **caractérisée en ce que** la poche de recueil (1) comprend deux orifices de sortie (16, 17) prévus respectivement sur la partie supérieure et sur la partie inférieure de la poche (1), une première (18) et une deuxième (19) poches étant prévues pour être en communication fluidique par l'intermédiaire d'une tubulure (20, 21) avec respectivement l'un de ces orifices (16, 17).

7. Procédé de prélèvement de sang placentaire à l'aide d'une ligne de prélèvement du sang placentaire, comprenant une poche de recueil (1) en communication fluidique, par l'intermédiaire d'une première tubulure (2) associée à un orifice d'entrée (3) de la poche (1), avec au moins une aiguille de prélèvement (4, 5), ladite ligne comprenant en outre une poche (6) contenant une solution de rincage qui est connectée ou qui est destinée à être connectée par l'intermédiaire d'une deuxième tubulure (7) à ladite première tubulure (2), ledit procédé comprenant les étapes prévoyant de :
- dans le cas où elle n'est pas connectée de fabrication à la ligne, connecter la poche (6) contenant la solution de rinçage ;
- réaliser une première ponction d'une veine du cordon ombilical ou du
placenta (P) avec une aiguille (4) après l'expulsion du placenta P et, après séparation du cordon C de l'enfant ;
- recueillir du sang placentaire dans la poche de recueil (1);
- vider, par l'intermédiaire de l'aiguille piquée (4), la poche (6) contenant la solution de rinçage à l'intérieur du cordon et du placenta ;
- réaliser une deuxième ponction de la veine du cordon ou du placenta (P) avec la même ou avec une autre aiguille (4, 5) ;
- recueillir, dans la poche de recueil (1), du sang placentaire éventuellement additionné de solution de rinçage ;
- éventuellement introduire la solution anticoagulante et/ou de conservation dans la poche de recueil (1).

8. Procédé selon la revendication 7, **caractérisé en ce qu'**il comprend une étape ultérieure de désolidarisation de la poche de recueil (1) de la ligne de prélèvement.

9. Procédé selon la revendication 7 ou 8, **caractérisé en ce que** la poche de recueil est ensuite centrifugée de sorte à permettre la récupération notamment des cellules souches hématopoïétiques.

## Patentansprüche

1. Abnahmeleitung für Plazentablut, umfassend einen Sammelbeutel (1) in fluidischer Verbindung, mit Hilfe von einem ersten Rohr (2), das einer Eintrittsöffnung (3) des Beutels (1) zugeordnet ist, mit einer ersten (4) und einer zweiten (5) Abnahmenadel, wobei die Abnahmenadeln in fluidischer Verbindung mit jeweils einem dritten (13) und einem vierten (14) Rohr sind, wobei das dritte (13) und das vierte (14) Rohr mit Hilfe von einem Verbindungsstück (15) dem ersten Rohr (2) zugeordnet sind, wobei die Leitung **dadurch gekennzeichnet ist, dass** sie des Weiteren einen Beutel (6) umfasst, der eine Spüllösung enthält, der mit Hilfe von einem zweiten Rohr (7) mit dem ersten Rohr (2) verbunden ist oder der dazu vorgesehen ist, derart verbunden zu werden.

2. Abnahmeleitung nach Anspruch 1, **dadurch gekennzeichnet, dass** das erste (2) und das zweite (7) Rohr mit Hilfe von einem ersten Verbindungsstück (8) verbunden sind.

3. Abnahmeleitung nach einem der Ansprüche 1 bis 2, **dadurch gekennzeichnet, dass** die Rohre jeweils Auswahlmittel zum Öffnen/Schließen der fluidischen Verbindung über das Rohr umfassen.

4. Abnahmeleitung nach einem der Ansprüche 1 bis 3, **dadurch gekennzeichnet, dass** der Sammelbeutel (1) eine Antikoagulanz- und/oder Konservierungslösung umfasst.

5. Abnahmeleitung nach einem der Ansprüche 1 bis 3, **dadurch gekennzeichnet, dass** sie des Weiteren einen Beutel (11) umfasst, der eine Antikoagulanz- und/oder Konservierungslösung enthält, wobei der Beutel (11) mit Hilfe von einem dritten Verbindungsstück (12), das unweit einer Abnahmenadel (4, 5) vorgesehen ist, auf dem ersten (2) oder auf dem vierten (14) Rohr verbunden ist.

6. Abnahmeleitung nach einem der Ansprüche 1 bis 5, **dadurch gekennzeichnet, dass** der Sammelbeutel (1) zwei Austrittsöffnungen (16, 17) umfasst, die jeweils am oberen Teil und am unteren Teil des Beutels (1) vorgesehen sind, wobei ein erster (18) und ein zweiter (19) Beutel vorgesehen, um in fluidischer Verbindung mit Hilfe von einem Rohr (20, 21) mit jeweils einer dieser Öffnungen (16, 17) zu sein.

7. Verfahren zur Abnahme von Plazentablut mit Hilfe von einer Abnahmeleitung für Plazentablut, umfassend einen Sammelbeutel (1) in fluidischer Verbindung, mit Hilfe von einem ersten Rohr (2), das einer Eintrittsöffnung (3) des Beutels (1) mit zumindest einer Abnahmenadel (4, 5) zugeordnet ist, wobei die Leitung des Weiteren einen Beutel (6) umfasst, der eine Spüllösung enthält, der mit Hilfe von einem zweiten Rohr (7) mit dem ersten Rohr (2) verbunden ist oder der dazu vorgesehen ist, derart verbunden zu werden, wobei das Verfahren folgende Schritte umfasst:
- in dem Fall, wo es nicht mit der Leitung während der Herstellung verbunden ist, den Beutel (6), der die Spüllösung enthält, verbinden;
- eine erste Punktion einer Ader der Nabelschnur oder des Plazenta (P) mit einer Nadel (4), nach der Geburt der Plazenta und nach der Trennung der Schnur von dem Kind, realisieren;
- Plazentablut im Sammelbeutel (1) auffangen;
- mit Hilfe von der eingestochenen Nadel (4), den Beutel (6), der die Spüllösung enthält, in die Nabelschnur und den Plazenta leeren;
- eine zweite Punktion der Ader der Nabelschnur oder des Plazenta (P) mit derselben oder einer anderen Nadel (4, 5) realisieren;
- Plazentablut gegebenenfalls mit zugefügter Spüllösung, im Sammelbeutel (1) auffangen;
- gegebenenfalls die Antikoagulanz- und/oder Konservierungslösung in den Sammelbeutel (1) geben.

8. Verfahren nach Anspruch 7, **dadurch gekennzeichnet, dass** er einen späteren Schritt der Lösung des Sammelbeutels (1) von der Abnahmeleitung umfasst.

9. Verfahren nach Anspruch 7 oder 8, **dadurch gekennzeichnet, dass** der Sammelbeutel anschließend derart zentrifugiert wird, dass das Auffangen insbesondere der hämatopoietischen Stammzellen möglich ist.

## Claims

1. A placental blood collection line including a collection bag (1) in fluid communication, via a first tube (2) associated with an inlet port (3) of the bag (1), with first (4) and second (5) collection needles, said collection needles being in fluid communication with third (13) and fourth (14) tubes respectively, said third (13) and fourth (14) tubes being associated with the first tube (2) via a coupling (15), said line being **characterised in that** it further includes a bag (6) containing a rinsing Solution, which is connected or is intended to be connected to said first tube (2) via a second tube (7).

2. A blood collection line according to claim 1, **characterised in that** the first (2) and second (7) tubes are connected via a first coupling (8).

3. A blood collection line according to any of claims 1 to 2, **characterised in that** each of the tubes includes selective opening/closing means of the fluid communication through the tube.

4. A blood collection line according to any of claims 1 to 3, **characterised in that** the collection bag (1) includes an anticoagulant and/or preservative solution.

5. A blood collection line according to any of claims 1 to 3, **characterised in that** it further includes a bag (11) containing an anticoagulant and/or preservative solution, said bag (11.) being connected via third coupling (12) provided in the vicinity of a collection needle (4, 5) on the first (2) or fourth (14) tube.

6. A blood collection line according to any of claims 1 to 5, **characterised in that** the collection bag (1) includes two outlet ports (16, 17) provided on the upper portion and lower portion of the bag (1), respectively, first (18) and second (19) bags being provided to be in fluid communication, via a tube (20,21), with one of said ports (16, 17), respectively.

7. A method for collecting placental blood by using a placental blood collection line, including a collection bag (1) in fluid communication, via a first tube (2) associated with an inlet port (3) of the bag (1), with at least one collection needle (4, 5), said line further including a bag (6) containing a rinsing solution, which is connected or intended to be connected via a second tube (7) to said first tube (2), said method including the steps providing for:
- in the case where it is not connected to the line during manufacture, connecting the bag (6) containing the rinsing solution;
- making a first puncture of a vein of the umbilical cord or placenta (P) with a needle (4), after expulsion of the placenta P and after separation of the cord C from the child;
- collecting the placental b100d in the collection bag (1);
- emptying, via the inserted needle (4), the bag (6) containing the rinsing solution into the umbilical cord and placenta;
- making a second puncture of the vein of the umbilical cord or placenta (P) with the same or with another needle (4, 5);
- collecting, in the collection bag (1), placental blood optionally with added rinsing solution;
- optionally introducing the anticoagulant and/or preservative solution into the collection bag (1).

8. The method according to claim 7, **characterised in that** it further includes a subsequent step of detaching the collection bag (1) from the collection line.

9. The method according to claim or 8, **characterised in that** the collection bag is then centrifuged so as to enable recovery, in particular, of the hematopoietic stem cells.
